# EUROPEAN PATENT APPLICATION

(11) **EP 4 252 666 A1**
(43) Date of publication of application: **04.10.2023**
(21) Application number: 22184961.5
(22) Date of filing: 14.07.2022
(51) Int. Cl.: A61B 6/12, A61B 6/00

(54) **DETERMINING A VALUE OF A PHYSICAL PROPERTY OF A THROMBUS**

(30) Priority: 29.03.2022 US 202263324753 P
(71) Applicant: Koninklijke Philips N.V., 5656 AG Eindhoven (NL)
(72) Inventor: SALEHI, Leili, Eindhoven (NL); SINHA, Ayushi, Eindhoven (NL); KYNE, Sean, 5656AG Eindhoven (NL); FOTOUHI, Javad, 5656AG Eindhoven (NL); PAI RAIKAR, Vipul Shrihari, Eindhoven (NL); ERKAMP, Ramon Quido, Eindhoven (NL)
(74) Representative: Philips Intellectual Property & Standards

(57) **Abstract**

A computer-implemented method of determining a value of a physical property of a thrombus, is provided. The method includes: receiving X-ray image data comprising a temporal sequence of X-ray images representing an expansion of a stent of an intraluminal stent retriever device over the thrombus; determining the value of the physical property of the thrombus based on a rate of expansion of the stent in the temporal sequence of X-ray images; and outputting the value of the physical property.

## Description

### TECHNICAL FIELD

The present disclosure relates to determining a value of a physical property of a thrombus. A computer-implemented method, a computer program product, and a system, are disclosed.

### BACKGROUND

A thrombus, or clot, is a blockage in a blood vessel. A thrombus may occur in a vein or in an artery. In the former case, i.e. a venous thrombus, blood becomes congested, leading to swelling and fluid congestion. In the latter case, i.e. an arterial thrombus, the supply of blood is restricted, leading to a condition known as ischemia, which risks damage to tissue supplied by the artery. In both cases, a portion of the thrombus can also break-away as an embolus. The embolus can become lodged elsewhere in the body and form an embolism that likewise blocks a blood vessel. Thromboses may occur in various parts of the body, including in the heart and the brain, where their effects can be severe unless treated quickly. In the brain, for example, a thrombus, or an embolism, can lead to conditions such as (ischemic) stroke.

Various intraluminal treatment devices are available for treating thromboses. These include mechanical thrombectomy devices, and which are used to remove a thrombus from a blood vessel. At present, there are two main groups of mechanical thrombectomy devices: aspiration catheters, and stent retrievers. Aspiration catheters typically include a delivery catheter that is used to deliver an irrigation fluid to the thrombus, and an extraction catheter that is used to extract the irrigation fluid, together with broken-up pieces of the thrombus. In-use, an aspiration catheter is positioned close to the clot, and at which position the aspiration takes place, resulting in the broken pieces of the clot being extracted from the body. Stent retrievers typically include an expandable wire mesh tube, i.e. stent, that is designed to remove the clot in one piece. Prior to its deployment, the wire mesh tube is housed in a delivery catheter. The delivery catheter is positioned such that it overlaps the clot, the delivery catheter is then withdrawn such that the wire mesh tube expands out of the distal end of the delivery catheter. As the wire mesh tube expands, it captures the clot. The wire mesh tube is then withdrawn into the delivery catheter and removed from the body.

Stent retriever "SR" thrombectomy is currently the first-line treatment for acute stroke due to intracranial large vessel occlusion "LVO" in anterior cerebral circulation. However, stent retriever thrombectomy procedures may fail for various reasons. These include anatomical challenges (e.g., a tortuous arterial tree from the aortic arch to a target occlusion site), large quantity of clots, tandem occlusion, clot characteristics (e.g. fresh versus organized clots), different pathomechanisms (embolic versus non-embolic occlusion), etc. The pathomechanism (embolic versus non-embolic) of an acute LVO or the physical properties of the clot (soft versus hard/organized) can play a key role in the response to a stent retriever. An organized (hard, fibrin-rich) clot is more resilient and less sticky than a fresh (soft, red blood cell-rich) clot, which results in less engagement with a stent retriever and can lead to a part or all of the clot missing during retrieval by a stent retriever, especially in the case of a tortuous arterial tree.

Furthermore, an organized clot can cause tension in the stent retriever-deployed segment of the parent artery, which can induce an arterial spasm. Such effects increase the probability of stent retriever failure. For successful recanalization of an LVO due to an organized clot, the first and simplest option is the intra-arterial administration of a vasodilator, which releases the tension of the stent retriever deployed arterial segment, thereby increasing the vessel diameter to ease clot retrieval. The second option is the intra-arterial administration of a clot-dissolving agent, tissue plasminogen activator (tPA), in order to soften the clot prior to retrieval. Another option is the simultaneous utilization of a stent retriever and an aspiration or intermediate catheter. A stent retriever and aspiration thrombectomy can be performed sequentially or simultaneously. This helps by preventing the engaged clots from being lost upon retraction of the stent retriever back through the tortuous or stenotic segment of the arterial tree. If the organized clot is still occluding the vessel after the simultaneous utilization of a stent retriever and an intermediate catheter, permanent stenting can be considered as a final action.

Another factor that increases the risk of stent retriever failure is the damage of the atheromatous surface by the stent if an acute LVO is due to intracranial atherosclerotic stenosis (ICAS) thrombo-occlusion. In this case, the use of a stent retriever can result in further platelet activation, leading to repeated re-occlusion in the future. To prevent repeated re-occlusion in the case of an LVO due to ICAS, inhibition of the platelet function can play a key role. Therefore, the first option is to administer a glycoprotein IIb/IIIa inhibitor, which inactivates platelets and thereby prevents repeated re-occlusion, as discussed in a document by Kim, B. M. "Causes and Solutions of Endovascular Treatment Failure.," J. stroke, vol. 19, no. 2, pp. 131-142, 2017.

The current workflow in a stent retriever thrombectomy procedure is to retrieve the stent between three and eight minutes after stent deployment. However, the chance of a successful clot retrieval in the first pass for hard/ organized clots is lower than that for soft clots. Some physicians may perform multiple rounds of stent retriever thrombectomy to retrieve the entire clot. Several alternative workflows such as those discussed above have also been suggested for treating organized clots. However, it has been reported in a document by Zaidat, O. O. et al., "First pass effect: A new measure for stroke thrombectomy devices," Stroke, vol. 49, no. 3, pp. 660-666, 2018, that there is a correlation between stent retriever procedures that are completed in a single pass and positive outcome. It has also been reported in a document by Ng, P. P. et al., "Intraprocedural predictors of post-stent retriever thrombectomy subarachnoid hemorrhage in middle cerebral artery stroke," J. Neurointerv. Surg., vol. 11, no. 2, pp. 123-126, 2019, that more than two rounds of SR can increase the risk of post-procedural subarachnoid hemorrhage "SAH". The same study shows a higher risk of post-procedural SAH in the patients who had the stent positioned more than 2 cm distal to the thrombus in their mid-cerebral arteries.

Thus, from the above discussion, it may be appreciated that the removal of a thrombus by mechanical thrombectomy is a challenging procedure, and that a knowledge of the physical properties of a thrombus would be useful in helping a physician determine the course of action in its extraction.

### SUMMARY

According to one aspect of the present disclosure, a computer-implemented method of determining a value of a physical property of a thrombus, is provided. The method includes:
receiving X-ray image data comprising a temporal sequence of X-ray images representing an expansion of a stent of an intraluminal stent retriever device over the thrombus;
determining the value of the physical property of the thrombus based on a rate of expansion of the stent in the temporal sequence of X-ray images; and
outputting the value of the physical property.

The inventors have observed that the rate of the expansion of a stent over a thrombus is indicative of various physical properties of a thrombus, including the dimensions of the thrombus, e.g. its length, diameter, and volume, and also the composition of the thrombus, e.g. soft versus hard, or organized. These physical properties affect the choices made by a physician on how to extract the thrombus. Thus, by using the above method, the physical properties of the thrombus may be determined during a stent retriever procedure, and consequently used by a physician to determine the course of action in extracting the thrombus.

Further aspects, features, and advantages of the present disclosure will become apparent from the following description of examples, which is made with reference to the accompanying drawings.

### BRIEF DESCRIPTION OF THE DRAWINGS

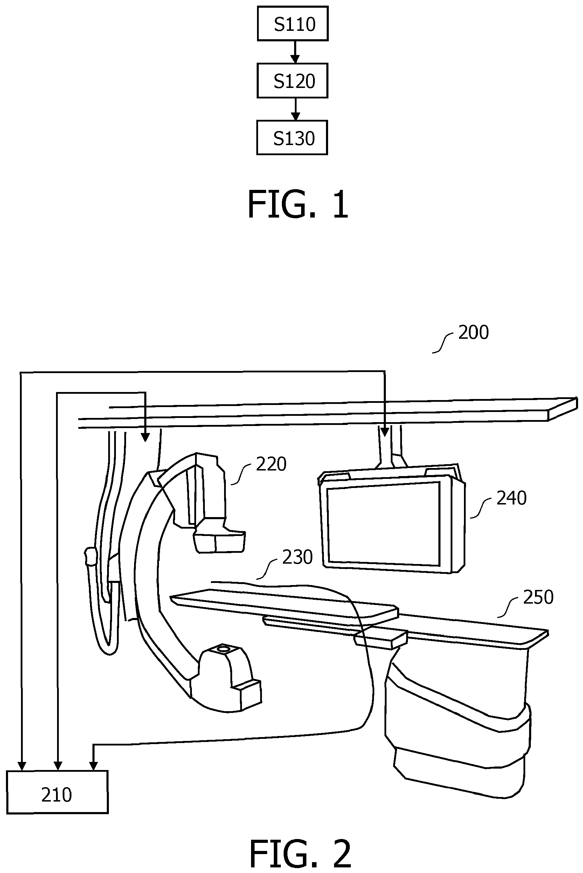

Fig. 1 is a flowchart illustrating an example of a method of determining a value of a physical property of a thrombus, in accordance with some aspects of the present disclosure.
Fig. 2 is a schematic diagram illustrating an example of a system 200 for determining a value of a physical property of a thrombus, in accordance with some aspects of the present disclosure.

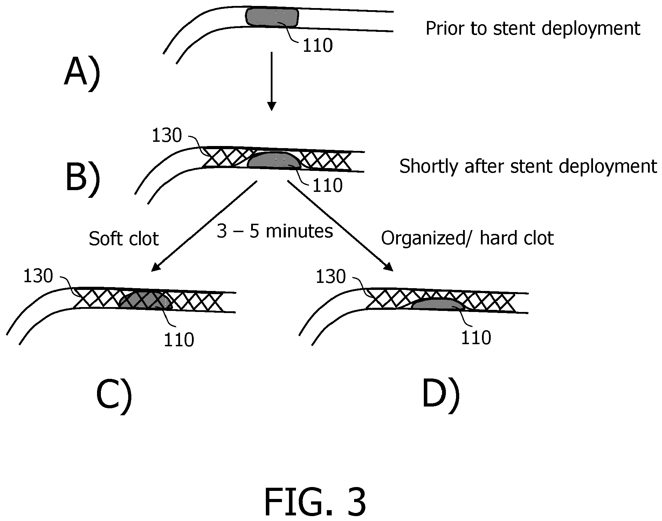

Fig. 3 is a schematic diagram illustrating an example of various stages in the expansion of a stent 130 of an intraluminal stent retriever device over a thrombus 110, in accordance with some aspects of the present disclosure.

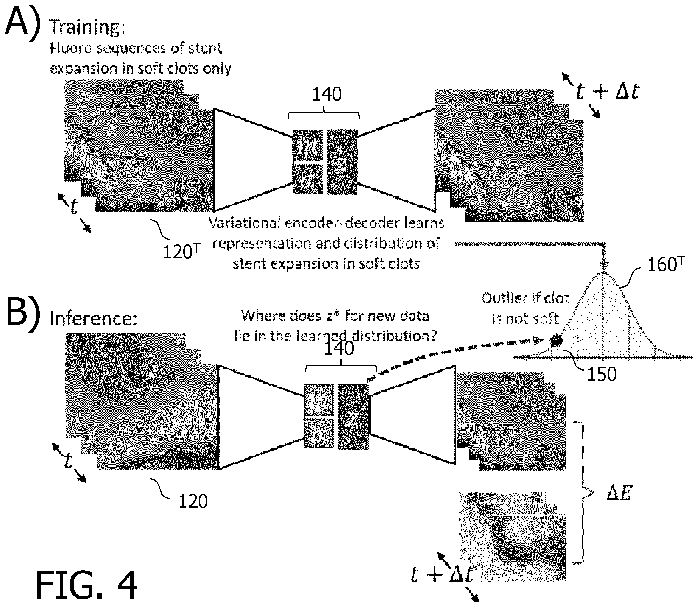

Fig. 4 is a schematic diagram illustrating an example of A) the training of a neural network 140 to generate latent space encodings of temporal sequences of X-ray images, and B) the performing of inference with a neural network 140 that is trained to generate latent space encodings of temporal sequences of X-ray images 120, in accordance with some aspects of the present disclosure.

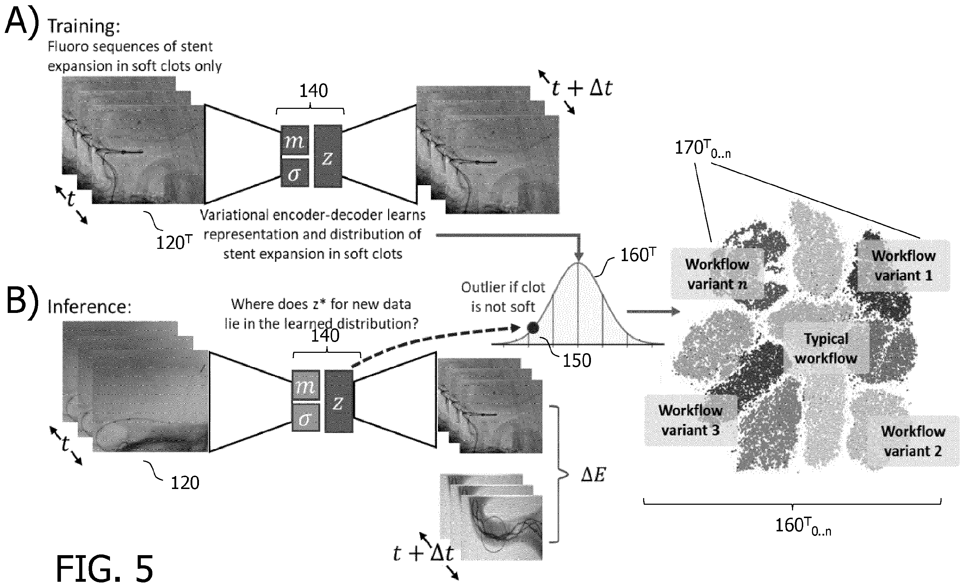

Fig. 5 is a schematic diagram illustrating an example of A) the training of a neural network 140 to generate latent space encodings of temporal sequences of X-ray images, and B) the performing of inference with a neural network 140 that is trained to generate latent space encodings of temporal sequences of X-ray images 120, and wherein the training data also includes workflow data, in accordance with some aspects of the present disclosure.

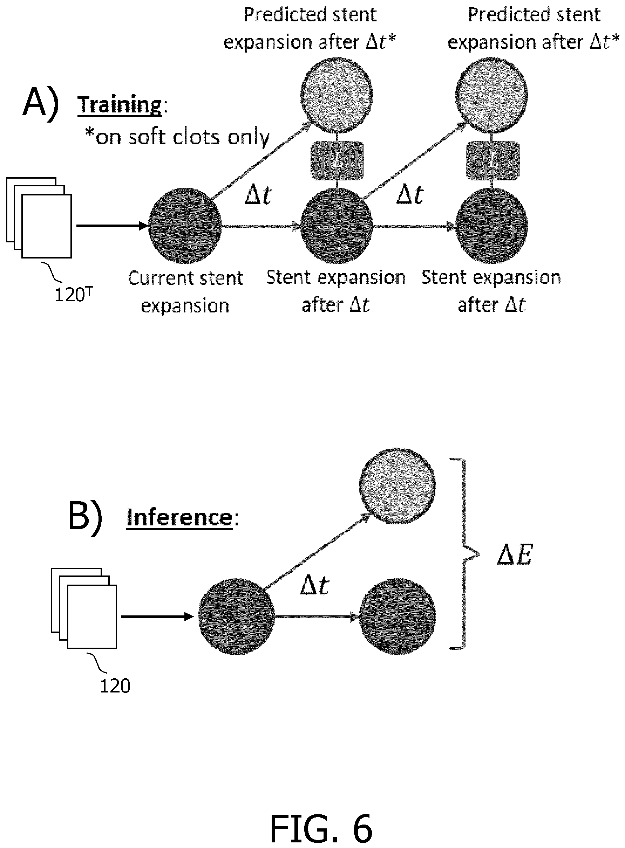

Fig. 6 is a schematic diagram illustrating an example of A) the training of a neural network 140 to predict the shape of a stent from a temporal sequences of X-ray images, and B) the performing of inference with a neural network 140 that is trained to predict the shape of a stent from a temporal sequences of X-ray images, in accordance with some aspects of the present disclosure.

### DETAILED DESCRIPTION

Examples of the present disclosure are provided with reference to the following description and figures. In this description, for the purposes of explanation, numerous specific details of certain examples are set forth. Reference in the specification to "an example", "an implementation" or similar language means that a feature, structure, or characteristic described in connection with the example is included in at least that one example. It is also to be appreciated that features described in relation to one example may also be used in another example, and that all features are not necessarily duplicated in each example for the sake of brevity. For instance, features described in relation to a computer implemented method, may be implemented in a computer program product, and in a system, in a corresponding manner.

In the following description, reference is made to computer-implemented methods that involve determining a value of a physical property of a thrombus. Reference is made to examples in which the thrombus is located in a blood vessel. In general, the thrombus may be located in a vein, or in an artery. In other words, the thrombus may be a venous thrombus, or alternatively it may be an arterial thrombus. It is to be appreciated that the thrombus may in general be located in any part of the body. For instance, the thrombus may be located in the brain, the heart, the lungs, or in a limb such as the leg, for example. In some examples the thrombus may be located in the brain and, the purpose of the treatment procedure may be for the treatment of (ischemic) stroke.

It is noted that the computer-implemented methods disclosed herein may be provided as a non-transitory computer-readable storage medium including computer-readable instructions stored thereon, which, when executed by at least one processor, cause the at least one processor to perform the method. In other words, the computer-implemented methods may be implemented in a computer program product. The computer program product can be provided by dedicated hardware, or hardware capable of running the software in association with appropriate software. When provided by a processor, the functions of the method features can be provided by a single dedicated processor, or by a single shared processor, or by a plurality of individual processors, some of which can be shared. The functions of one or more of the method features may for instance be provided by processors that are shared within a networked processing architecture such as a client/server architecture, a peer-to-peer architecture, the Internet, or the Cloud.

The explicit use of the terms "processor" or "controller" should not be interpreted as exclusively referring to hardware capable of running software, and can implicitly include, but is not limited to, digital signal processor "DSP" hardware, read only memory "ROM" for storing software, random access memory "RAM", a non-volatile storage device, and the like. Furthermore, examples of the present disclosure can take the form of a computer program product accessible from a computer-usable storage medium, or a computer-readable storage medium, the computer program product providing program code for use by or in connection with a computer or any instruction execution system. For the purposes of this description, a computer-usable storage medium or a computer readable storage medium can be any apparatus that can comprise, store, communicate, propagate, or transport a program for use by or in connection with an instruction execution system, apparatus, or device. The medium can be an electronic, magnetic, optical, electromagnetic, infrared, or a semiconductor system or device or propagation medium. Examples of computer-readable media include semiconductor or solid state memories, magnetic tape, removable computer disks, random access memory "RAM", read-only memory "ROM", rigid magnetic disks and optical disks. Current examples of optical disks include compact disk-read only memory "CD-ROM", compact disk-read/write "CD-R/W", Blu-Ray^{™} and DVD.

As mentioned above, the removal of a thrombus by mechanical thrombectomy is a challenging procedure, and a knowledge of the physical properties of a thrombus would be useful in helping a physician determine the course of action in its extraction.

Fig. 1 is a flowchart illustrating an example of a method of determining a value of a physical property of a thrombus, in accordance with some aspects of the present disclosure. With reference to Fig. 1, the method includes:
receiving S110 X-ray image data comprising a temporal sequence of X-ray images 120 representing an expansion of a stent 130 of an intraluminal stent retriever device over the thrombus 110;
determining S120 the value of the physical property of the thrombus 110 based on a rate of expansion of the stent 130 in the temporal sequence of X-ray images 120; and
outputting S130 the value of the physical property.

The inventors have observed that the rate of the expansion of a stent over a thrombus is indicative of various physical properties of a thrombus, including the dimensions of the thrombus, e.g. its length, diameter, and volume, and also the composition of the thrombus, e.g. soft versus hard, or organized. These physical properties affect the choices made by a physician on how to extract the thrombus. Thus, by using the above method, the physical properties of the thrombus may be determined during a stent retriever procedure, and consequently used by a physician to determine the course of action in extracting the thrombus.

The method described above with reference to Fig. 1, may also be implemented by the system illustrated in Fig. 2, which is a schematic diagram illustrating an example of a system 200 for determining a value of a physical property of a thrombus, in accordance with some aspects of the present disclosure. Thus, operations described in relation to the flowchart illustrated in Fig. 1, may also be performed by the system 200 illustrated in Fig. 2, and vice versa.

With reference to Fig. 1, in the operation S110, X-ray image data is received. The X-ray image data includes a temporal sequence of X-ray images 120 representing an expansion of a stent 130 of an intraluminal stent retriever device over a thrombus 110.

In general, the X-ray image data that is received in the operation S110 may be generated by a projection X-ray imaging system. Projection X-ray imaging systems typically include a support arm such as a so-called "C-arm", or an "O-arm", that supports an X-ray source-detector arrangement. Projection X-ray imaging systems may alternatively include a support arm with a different shape to these examples. Projection X-ray imaging systems typically generate projection X-ray images with the support arm held in a static position with respect to an imaging region during the acquisition of image data. The temporal sequence of X-ray images 120 that is received in the operation S110 may be acquired periodically, i.e. at regular intervals, or intermittently, i.e. at irregular intervals. The temporal sequence of X-ray images 120 may be fluoroscopic, i.e. live images. This allows the operation of determining S120 the value of the physical property of the thrombus 110 to be carried-out in real-time. The X-ray image data may be generated by the projection X-ray imaging system 220 illustrated in Fig. 2, for example. By way of an example, the X-ray image data may be generated by the Philips Azurion 7 X-ray imaging system marketed by Philips Healthcare, Best, The Netherlands.

The X-ray image data that is received in the operation S110 includes a temporal sequence of X-ray images 120 representing an expansion of a stent 130 of an intraluminal stent retriever device over a thrombus 110. In this regard, the temporal sequence may be generated during a stent retriever procedure to extract a thrombus from a vein. As mentioned above, a stent retriever typically includes an expandable wire mesh tube, i.e. stent, that is designed to remove the clot in one piece. Prior to its deployment, the wire mesh tube is housed in a delivery catheter. The delivery catheter is positioned such that it overlaps the clot, the delivery catheter is then withdrawn such that the wire mesh tube expands out of the distal end of the delivery catheter. As the wire mesh tube expands, it captures the clot. The wire mesh tube is then withdrawn into the delivery catheter and removed from the body. The temporal sequence of X-ray images 120 represents the stent and the thrombus during at least some of the period in which the wire mesh tube, or stent, is expanding.

The X-ray image data that is received in the operation S110 may be received from a projection X-ray imaging system, such as the projection X-ray imaging system 220 illustrated in Fig. 2, or from a computer readable storage medium, or from the Internet or the Cloud, for example. The X-ray image data may be received by the one or more processors 210 illustrated in Fig. 2. The X-ray image data may be received via any form of data communication, including wired, optical, and wireless communication. By way of some examples, when wired or optical communication is used, the communication may take place via signals transmitted on an electrical or optical cable, and when wireless communication is used, the communication may for example be via RF or optical signals.

In the operation S120, the value of the physical property of the thrombus 110 is determined based on a rate of expansion of the stent 130 in the temporal sequence of X-ray images 120. In this regard, the physical property may for example be a dimension of the thrombus, such as for example its length, or its diameter, or its volume, or a composition of the thrombus, such as for example whether it is soft versus hard, or organized.

Fig. 3 is a schematic diagram illustrating an example of various stages in the expansion of a stent 130 of an intraluminal stent retriever device over a thrombus 110, in accordance with some aspects of the present disclosure. Illustration A in Fig. 3 represents the thrombus prior to the introduction of the stent retriever "SR" into the vessel. In illustration A, the thrombus 110 completely blocks the vessel. Illustration B in Fig. 3 represents the thrombus and the stent of the stent retriever device shortly after deployment of the stent from the stent retriever. In illustration B, the stent has been positioned such that it overlaps with the thrombus, and the stent has started to expand over the thrombus. The stent squeezes the thrombus transversely with respect to the vessel axis, and the outer surface of the stent conforms to the outer surface of the thrombus. At approximately three to five minutes after deployment of the stent, illustration C represents the stent and the thrombus for a soft clot. In this example, the stent has cut through the soft clot, completely enveloping it. At approximately three to five minutes after stent deployment, illustration D represents the stent and the thrombus for a hard, or organized clot. In this example, the stent has further squeezed the thrombus transversely with respect to the vessel axis as compared to illustration B, and the outer surface of the stent still conforms to the outer surface of the thrombus. After a further period of time, and depending on physical properties of the thrombus such as its size and hardness, the stent for the hard clot might remain in the same or a similar state of expansion as in illustration D, or it might cut through some or all of the clot, thereby partially or completely enveloping it. As seen in the illustrations C and D in particular, a distinction between a soft clot and a hard clot may therefore be made based on the rate of expansion of the stent; the rate of expansion of the stent being higher for the soft clot in illustration C, than for the hard clot as in illustration D.

The rate of expansion of the stent also provides information on other physical properties of the clot than the hardness. For instance, a length of the stent may be determined based on the rate of expansion of the stent at multiple positions along its length over time. In the initial period of the stent's expansion, i.e. in the time interval between the stent's deployment and the stage represented by illustration B, the stent squeezes the thrombus in a direction perpendicular to the axis of the vessel, and the outer surface of the stent conforms to the outer surface of the thrombus. During this initial period, a length of the thrombus may be determined based on the position along the length of the stent at which a rate of the stent's expansion undergoes a significant reduction. These positions may correspond regions in which the outer surface of the stent contacts the outer surface of the thrombus. Therefore, a length of the clot may be determined based on the rate of expansion of the stent along its length. Since the stent is more-easily discernible in X-ray images than the thrombus, this technique may provide an accurate method of determining the length of the clot.

A diameter, and a volume of the thrombus may also be determined based on the rate of expansion of the stent along its length. For example, a model representing the rate of expansion of the stent along its length may be generated for thrombi having different diameters, or volumes. The model may then be used to associate the diameter and volume of the thrombus with the rate of expansion of the stent along its length. The model may also be used to generate a lookup table of values of the rate of expansion of the stent along its length for different values of the diameter, or volume. In use, the diameter and volume of the thrombus may be determined from the lookup table using the detected values of the rate of expansion of the stent along its length.

The rate of expansion of the stent 130 may be determined in the operation S120 using various techniques. For instance, image analysis techniques may be used to determine the rate of expansion of the stent at one or more positions along its length in the temporal sequence of X-ray images, and a physical property of the thrombus may be determined from the rate of expansion as described above. By registering the images to each other using e.g. image intensities or the ends of the stent, changes in the shape of the stent in the images may be determined, and these changes used to calculate the rate of expansion using the time interval between the images. The rate of expansion can then be used to determine the value of the physical property of the thrombus. In other examples, a neural network is used to determine the value of the physical property of the thrombus 110 in the operation S120, as described in more detail below.

In the operation S130, the value of the physical property is outputted. The value of the physical property may be outputted in various ways, including to a computer readable storage medium, or to a display device, such as the monitor 240 illustrated in Fig. 2. By outputting the value of the physical property, a physician may use this information to determine the course of action in treating the thrombus.

As mentioned above, in some examples, a neural network is used to determine the value of the physical property of the thrombus 110 in the operation S120. Examples of neural networks for this purpose are described with reference to Fig. 4 and Fig. 5.

Fig. 4 is a schematic diagram illustrating an example of A) the training of a neural network 140 to generate latent space encodings of temporal sequences of X-ray images, and B) the performing of inference with a neural network 140 that is trained to generate latent space encodings of temporal sequences of X-ray images 120, in accordance with some aspects of the present disclosure. The example neural network 140 illustrated in Fig. 4 has a variational autoencoder architecture. The neural network 140 may alternatively have another architecture, such as a convolutional neural network "CNN" architecture, or a recurrent neural network "RNN" architecture with unidirectional or bidirectional long short-term memory "LSTM" architecture, etc. With reference to Fig. 4A, during training, temporal sequences of training X-ray images 120^{T} that represent an expansion of a stent of an intraluminal stent retriever device over a thrombus, are inputted into the variational autoencoder neural network 140, and the neural network is trained to generate latent space encodings, z, that represent the inputted temporal sequences. The variational autoencoder is trained by forcing its decoder to re-generate the temporal sequences of training X-ray images 120^{T} that are inputted into its encoder, from the latent space encodings, z, that are generated by its encoder. Multiple temporal sequences of training X-ray images are used to train the neural network 140. During training, the variational autoencoder also learns a distribution 160^{T} of the latent space encodings of the temporal sequences of training X-ray images 120^{T}. This distribution is represented in Fig. 4 as a normal distribution 160^{T} having a mean, *m* and variance, s. The training data that is used to train the neural network 140 is selected such that it represents a thrombus with a physical property having a known value. The training data may for instance represent a soft clot. In so doing, the neural network 140 learns to generate a latent space encodings, z, of inputted temporal sequences of images, as well as a distribution 160^{T} of the latent space encodings, for thrombi having the known value of the physical property, e.g. for thrombi that are soft.

With reference to Fig. 4B, at inference, new temporal sequences of X-ray images 120 are inputted into the trained neural network 140. The trained neural network 140 generates a latent space encoding 150, z of the inputted images in each new sequence. The position of the latent space encoding 150 for the new temporal sequence, is then determined with respect to the distribution 160^{T} of latent space encodings that were generated from the temporal sequences of training X-ray images 120^{T}. If the clot represented in the new temporal sequence of X-ray images 120 is of a similar type as the clot in the temporal sequences of training X-ray images 120^{T}, e.g. if the clot is a soft clot, the latent space encoding 150 will be close to the mean of the distribution 160^{T} of latent space encodings that were generated from the temporal sequences of training X-ray images 120^{T}. In other words, the latent space encoding 150 will be an inlier to the distribution 160^{T}. By contrast, if the clot represented in the new temporal sequence of X-ray images 120 is a different type of clot to the clot in the temporal sequences of training X-ray images 120^{T}, e.g. if the clot is a hard clot, the latent space encoding 150 will be distant from the mean of the distribution 160^{T} of latent space encodings that were generated from the temporal sequences of training X-ray images 120^{T}. In other words, the latent space encoding 150 will be an outlier to the distribution 160^{T}. In the example illustrated in Fig. 4, the latent space encoding 150 is an outlier to the distribution 160^{T} that was generated using temporal sequences for soft clots, and so it is concluded that the clot in the new temporal sequence of X-ray images 120 is a hard clot.

More generally, the value of a physical property of the thrombus 110 may be determined using the neural network illustrated in Fig. 4 based on a position of the latent space encoding 150, z of the inputted images, with respect to a distribution 160^{T} of latent space encodings generated from temporal sequences of training X-ray images 120^{T} representing an expansion of a stent over thrombi having a known value for the physical property.

In the above example, it is noted that the decoder portion of the neural network that reconstructs the inputted images during training, i.e. the portion to the right-hand side of Fig. 4A and Fig. 4B, is not essential for performing inference. This is because in this example, the value of the physical property of the thrombus 110 is determined using the latent space encoding 150, z of the inputted images, and this does not rely on the presence of a reconstructed image. In this example, the decoder portion is simply used to train the neural network. In another example described below, the decoder portion of the neural network is used at inference and the neural network determines the value of the physical property of the thrombus 110 based on a difference DE between a predicted shape of the stent, and an actual shape of the stent in the inputted images.

In the above example, "clot hardness" was used as an example of the physical property of the thrombus for which the training data was selected, and the known value was "soft". However, by alternatively, or additionally, training the neural network 140 using training data that represents other known values of a physical property of the thrombus, and determining, at inference, the position of the latent space encoding 150, z of a new temporal sequence of X-ray images 120 with respect to each of these distributions, the neural network 140 may also be used to determine how close the latent space encoding of the new temporal sequence of X-ray images 120 is, to the mean values of the distributions for these other known values of physical properties. In so doing, the neural network 140 may be trained, and used to determine the value of other physical properties of the thrombus 110.

Thus, with reference to the method illustrated in Fig. 1, in one example, the operation of determining S120 the value of the physical property of the thrombus 110, may include:
inputting a plurality of images from the temporal sequence into a neural network 140;
generating, using the neural network 140, a latent space encoding 150, z of the inputted images; and
determining the value of the physical property of the thrombus 110 based on a position of the latent space encoding 150, z of the inputted images, with respect to a distribution 160^{T} of latent space encodings generated from temporal sequences of training X-ray images 120^{T} representing an expansion of a stent over thrombi having a known value for the physical property; and
wherein the neural network 140 is trained to generate the latent space encoding 150, z of the inputted images using training data comprising the temporal sequences of training X-ray images 120^{T}, and wherein each sequence represents an expansion of a stent of an intraluminal stent retriever device over a thrombus having a known value for the physical property.

In the above method, the position of the latent space encoding 150, z, of the inputted images with respect to the distribution 160^{T} of latent space encodings generated from temporal sequences of training X-ray images 120^{T}, can be determined by calculating a distance between the position of the latent space encoding 150, z of the inputted images and a mean, or centroid of the distribution. This distance can be measured using a function such as the Euclidean distance or the geodesic distance, for example. This distance provides an analogue measure of how close the physical property of the thrombus represented in the inputted images is to the known value of the physical property. This measure may be represented on a continuous scale as a fraction or percent representing how close the physical property of the thrombus represented in the inputted images is to the known value of the physical property. Alternatively, by digitizing this distance using a threshold value, a binary classification, for example a hard composition versus a soft composition, of the physical property may be provided. The threshold value may for example be determined based on the standard deviation, s, of the distribution 160^{T} of latent space encodings. For instance, if the distance is less than Is for a new temporal sequence then the new temporal sequence may be classified with the known value of the physical property from the training data.

The neural network described with reference to Fig. 4 may be trained to generate the latent space encoding 150, z of the inputted images, by:
receiving training data, including a plurality of temporal sequences of training X-ray images 120^{T}, and wherein each temporal sequence represents an expansion of a stent of an intraluminal stent retriever device over a thrombus having a known value for the physical property;
inputting the training data into the neural network 140; and
for each of a plurality of the inputted training X-ray images in a temporal sequence:
   generating a latent space encoding z of the inputted training X-ray image, using the neural network 140;
   reconstructing the inputted training X-ray image from the latent space encoding z, using the neural network 140; and
   adjusting parameters of the neural network 140 based on a difference between the inputted training X-ray image and the reconstructed inputted training X-ray image; and
   repeating the generating, the reconstructing, and the adjusting, until a stopping criterion is met.

In general, the training of a neural network involves inputting a training dataset into the neural network, and iteratively adjusting the neural network's parameters until the trained neural network provides an accurate output. Training is often performed using a Graphics Processing Unit "GPU" or a dedicated neural processor such as a Neural Processing Unit "NPU" or a Tensor Processing Unit "TPU". Training often employs a centralized approach wherein cloud-based or mainframe-based neural processors are used to train a neural network. Following its training with the training dataset, the trained neural network may be deployed to a device for analyzing new input data during inference. The processing requirements during inference are significantly less than those required during training, allowing the neural network to be deployed to a variety of systems such as laptop computers, tablets, mobile phones and so forth. Inference may for example be performed by a Central Processing Unit "CPU", a GPU, an NPU, a TPU, on a server, or in the cloud.

The process of training the neural network 140 described above therefore includes adjusting its parameters. The parameters, or more particularly the weights and biases, control the operation of activation functions in the neural network. In supervised learning, the training process automatically adjusts the weights and the biases, such that when presented with the input data, the neural network accurately provides the corresponding expected output data. In order to do this, the value of the loss functions, or errors, are computed based on a difference between predicted output data and the expected output data. The value of the loss function may be computed using functions such as the negative log-likelihood loss, the mean absolute error (or L1 norm), the mean squared error, the root mean squared error (or L2 norm), the Huber loss, or the (binary) cross entropy loss. Other loss functions like the Kullback-Leibler divergence may additionally be used when training a variational autoencoder to ensure that the distribution 160^{T} of latent space encodings generated from temporal sequences of training X-ray images 120^{T} is similar to a standard Gaussian distribution with mean 0 and standard deviation of 1. During training, the value of the loss function is typically minimized, and training is terminated when the value of the loss function satisfies a stopping criterion. Sometimes, training is terminated when the value of the loss function satisfies one or more of multiple criteria.

Various methods are known for solving the loss minimization problem such as gradient descent, Quasi-Newton methods, and so forth. Various algorithms have been developed to implement these methods and their variants including but not limited to Stochastic Gradient Descent "SGD", batch gradient descent, mini-batch gradient descent, Gauss-Newton, Levenberg Marquardt, Momentum, Adam, Nadam, Adagrad, Adadelta, RMSProp, and Adamax "optimizers" These algorithms compute the derivative of the loss function with respect to the model parameters using the chain rule. This process is called backpropagation since derivatives are computed starting at the last layer or output layer, moving toward the first layer or input layer. These derivatives inform the algorithm how the model parameters must be adjusted in order to minimize the error function. That is, adjustments to model parameters are made starting from the output layer and working backwards in the network until the input layer is reached. In a first training iteration, the initial weights and biases are often randomized. The neural network then predicts the output data, which is likewise, random. Backpropagation is then used to adjust the weights and the biases. The training process is performed iteratively by making adjustments to the weights and biases in each iteration. Training is terminated when the error, or difference between the predicted output data and the expected output data, is within an acceptable range for the training data, or for some validation data. Subsequently the neural network may be deployed, and the trained neural network makes predictions on new input data using the trained values of its parameters. If the training process was successful, the trained neural network accurately predicts the expected output data from the new input data.

In general, the training data used in the training method described above with reference to Fig. 4 may be extracted from procedural records of historic mechanical thrombectomy procedures in which an intraluminal stent retriever device was used. The temporal sequences of training X-ray images 120^{T} typically form part of these procedural records. Temporal sequences of training X-ray images 120^{T} from tens, hundreds, or thousands, or more, of such procedures may be used to train the neural network 140. The training data may represent patients having different ages, gender, body mass index, and so forth. The known physical property of the thrombus represented in the training data may be a known value of a dimension of the thrombus, or a known composition of the thrombus, as described above. A dimension of the thrombus may be known by calculating its value from the training X-ray images using a scaling factor that is determined based on the geometry of the X-ray source, the X-ray detector, and the relative positions of the X-ray source and X-ray detector respective the thrombus, or alternatively it may be determined by measuring the extracted clot during post-procedural histological analysis. Such information is typically stored in the procedural records. The composition of the thrombus may likewise be determined from a histological analysis of the extracted clot, and this information is also typically stored in the procedural records. The physical properties of the thrombus may alternatively be determined from image data, such as magnetic resonance image data representing the thrombus, or from CT image data representing the thrombus, or from intravascular ultrasound "IVUS" image data representing the thrombus.

The neural network 140 may alternatively be trained using training data in which thrombi having known values of another physical property are represented. In so doing, the neural network may be used to determine how close a physical property of a thrombus in a new temporal sequence is to other types of physical property than its dimensions or its composition, as described in the above examples.

In one example, the neural network described with reference to Fig. 4 is trained such that it places higher emphasis on data within a defined region of interest than outside the region of interest. In this example, the region of interest includes the stent and the thrombus. The region of interest may be defined manually or automatically in the training data, i.e. in the temporal sequences of training X-ray images 120^{T}. For example, a user may define a region of interest manually in the training X-ray images 120^{T} by providing user input via a user input device such as a mouse operating in combination with a graphical user interface "GUI" that displays the training X-ray images 120^{T}. The region of interest may be defined by the user using a bounding box, for example. Alternatively, the region of interest may be determined automatically. For instance, the region of interest may be defined in the training X-ray images 120^{T} using a trained neural network, or a feature detection algorithm. Thus, in one example, the neural network 140 is trained to generate the latent space encoding 150, z of the inputted images, by further:
receiving input indicative of a region of interest in the temporal sequences of training X-ray images, the region of interest comprising the stent and the thrombus; and
wherein the adjusting parameters of the neural network based on a difference between the inputted training X-ray image and the reconstructed inputted training X-ray image within a region of interest; is performed by applying a weighting to the difference, and wherein a relatively higher weighting is applied to portions of the temporal sequences of training X-ray images within the region of interest, and a relatively lower weighting is applied to portions of the temporal sequences of training X-ray images outside the region of interest.

By using the region of interest to train the neural network in this manner, the neural network may be trained to generate latent space encodings that are more specific to the region of interest. Thus, the neural network may provide a more accurate determination of the value of the physical property of the thrombus 110.

Various additional data may also be used to train the neural network 140, as described in the examples below.

As outlined above, the process of extracting a thrombus is complex, and the workflow steps that are taken by a physician during its extraction depend in-part on the physical properties of the thrombus. For instance, an organized (hard, fibrin-rich) clot is more resilient and less sticky than a fresh (soft, red blood cell-rich) clot, which results in less engagement with a stent retriever and can lead to a part or all of the clot missing during retrieval by a stent retriever, especially in the case of a tortuous arterial tree. Consequently, the delay between deploying and retrieving a stent of a stent retriever device may vary depending on its composition. By way of another example, an organized clot can cause tension in the stent retriever-deployed segment of the parent artery, which can induce an arterial spasm. In this case, an intra-arterial vasodilator may be administered, or a stent retriever and an aspiration or intermediate catheter may be used. Permanent stenting using e.g. a balloon angioplasty device, may also be considered as a final action. A glycoprotein IIb/IIIa inhibitor may also be administered in order to inactivate platelets and thereby prevent repeated re-occlusion of the vessel.

A physician performing a mechanical thrombectomy procedure is therefore faced with numerous choices of workflow steps such as "wait X minutes between stent deployment and retrieval", "inject vasodilator", "use stent retriever in combination with aspiration catheter/ intermediate catheter", "insert permanent stent using balloon angioplasty procedure", "administer a glycoprotein IIb/IIIa inhibitor". Each of these steps may impact the success of the procedure. Other workflow steps that may also impact the success of the procedure, and which are also carried out during a mechanical thrombectomy procedure include steps such as "move the distal end of the stent retriever/ aspiration catheter to X centimeters from the clot", "increase aspiration by the aspiration catheter", "generate 3D CT image to detect the risk of SAH". These latter workflow steps may or may not depend on the physical properties of the thrombus. However, they may also impact the success of the procedure.

In some examples, the training data that is used to train the neural network described above with reference to Fig. 4, also includes workflow steps which were used to successfully treat the thrombus in the temporal sequences of training X-ray images. This data may be extracted from the procedural records of historic mechanical thrombectomy procedures. At inference, these workflow steps are used to provide recommendations for a subsequent workflow step(s) during a mechanical thrombectomy procedure.

In these examples, the training data that is used to train the neural network described with reference to Fig. 4, also includes workflow data for each temporal sequence of training X-ray images 120^{T}. The workflow data defines one or more subsequent workflow steps used to successfully treat the thrombus in the temporal sequence of training X-ray images. At inference, the method described with reference to Fig. 4 also includes:
determining a position of the latent space encoding 150, z of the inputted images, with respect to a distribution 160^{T} of the latent space encodings having common workflow data; and
outputting the common workflow data to provide one or more recommended subsequent workflow steps for treating the thrombus 110.

By providing such recommendations, a physician performing a mechanical thrombectomy procedure is provided with additional guidance that may be used to improve the success of the procedure.

The above method of recommending workflow steps stems from the insight that if the latent space encoding of the sequence of images from a current procedure, is similar to the latent space encodings of the sequences of images from the training data, then a successful workflow step that is taken in the procedures represented in the training data, may also yield a similar level of success in the current procedure. The above method may be visualized as follows. The temporal sequences of training X-ray images 120^{T} are each labelled with one or more subsequent steps that were used to successfully treat the thrombus in the sequence. The latent space encodings of the temporal sequences of training X-ray images 120^{T} form clusters in latent space according to the subsequent step(s) that were used to successfully treat the thrombus in each sequence. For instance, temporal sequences that share the common workflow step "wait three minutes between stent deployment and retrieval" may form one cluster in latent space. Temporal sequences that share the common workflow step "wait five minutes between stent deployment and retrieval" may form another cluster in latent space. At inference, the position of a latent space encoding of a new temporal sequence of X-ray images is determined with respect to each of these clusters. If the latent space encoding of the new temporal sequence is closer to the cluster "wait five minutes between stent deployment and retrieval", then this workflow step is outputted as the recommended subsequent workflow step for treating the thrombus. By contrast, if the latent space encoding of the new temporal sequence is closer to the cluster "wait three minutes between stent deployment and retrieval", then this workflow step is outputted. The position of a latent space encoding for a new temporal sequence of images may be determined with respect to a cluster by determining a distance between a centroid of the distribution of latent space encodings that form the cluster. The distance may be calculated in the same way as described above, i.e. by using a function such as the Euclidean distance or the geodesic distance, for example.

A related example is described with reference to Fig. 5, which is a schematic diagram illustrating an example of A) the training of a neural network 140 to generate latent space encodings of temporal sequences of X-ray images, and B) the performing of inference with a neural network 140 that is trained to generate latent space encodings of temporal sequences of X-ray images 120, and wherein the training data also includes workflow data, in accordance with some aspects of the present disclosure. Fig. 5 corresponds to Fig. 4, and additionally includes a depiction of multiple distribution 160^{T}_{0..n} of latent space encodings having common workflow data. Features in Fig. 5 that share the same labels as features in Fig. 4 perform the same function as described with reference to Fig. 4. In the example illustrated in Fig. 5, the latent space encodings of temporal sequences having common workflow steps 170^{T}_{0..n} form clusters, i.e. the distributions 160^{T}_{0..n}. By way of an example, the workflow step "Typical workflow" distribution may represent a cluster of latent space encodings for successful procedures in which the workflow step "inject vasodilator" was performed. The workflow step "Workflow variant 1" distribution may for instance represent a cluster of latent space encodings for successful procedures in which a different workflow step "use stent retriever in combination with aspiration catheter" was performed. The workflow step "Workflow variant 2" distribution may for instance represent a cluster of latent space encodings for successful procedures in which a different workflow step "insert permanent stent using balloon angioplasty procedure" was performed. At inference, the position of a latent space encoding for a new temporal sequence is determined with respect to the centroid of each of the distributions 160^{T}_{0..n} that have corresponding workflow steps 170^{T}_{0..n}. The workflow step corresponding to the distribution 160^{T}_{0..n} that has the closest centroid is then outputted as the recommended subsequent step.

Thus, in this example, the training data that is used to train the neural network 140 described with reference to Fig. 4, includes a plurality of temporal sequences of training X-ray images having different workflow data 170^{T}_{0..n}; and the operation of determining a position of the latent space encoding 150, z of the inputted images, with respect to a distribution 160^{T}_{0..n} of the latent space encodings having common workflow data, comprises determining the position of the latent space encoding 150, z of the inputted images, with respect to a centroid of each distribution 160 ^{T}_{0..n} of the latent space encodings having common workflow data. Furthermore, the operation of outputting the common workflow data to provide one or more recommended subsequent workflow steps for treating the thrombus 110, comprises outputting the common workflow data for the distribution 160^{T}_{0..n} having the nearest centroid to the position of the latent space encoding 150, z of the inputted images.

In these examples, the workflow step(s) may also include a success metric representing a probability of the one or more subsequent workflow steps resulting in a successful treatment of the thrombus 110. At inference, the success metric for the outputted workflow step(s) may also be outputted. The success metric may also be computed from the procedural records of historic mechanical thrombectomy procedures. For instance, the success metric may be estimated by computing, in some neighborhood of the position of the latent space encoding 150, z of the inputted images, the percentage of successful procedures in which the common workflow steps corresponding to the distribution 160^{T}_{0..n} that has the closest centroid were performed out of all successful procedures in the neighborhood of the position of the latent space encoding 150, z of the inputted images. Therefore, if the position of the latent space encoding 150, z of the inputted images is close to the centroid, the success metric computed may be higher. If the position of the latent space encoding 150, z of the inputted images is far away from the centroid and close to the edge of the distribution 160^{T}_{0..n}, then the success metric may be lower as the neighborhood of the position of the latent space encoding 150, z of the inputted images may overlap with a different distribution than the distribution that has the closest centroid. Alternatively, the success metric may be outputted directly by a neural network 140 or may be based on the reconstruction error DE between the predicted images, and the inputted images. By outputting the success metric, the method facilitates the physician to perform the recommended workflow step with confidence in its expected outcome. Thus in some examples, the workflow data may also include a success metric corresponding to the one or more subsequent workflow steps; the success metric representing a probability of the one or more subsequent workflow steps resulting in a successful treatment of the thrombus 110; and the operation of outputting the common workflow data, further comprises outputting the corresponding success metric.

In some examples, the operation of determining a position of the latent space encoding 150, z of the inputted images, with respect to a distribution 160 of the latent space encodings having common workflow data, may be carried out using a neural network. In these examples, the operation of determining a position of the latent space encoding 150, z of the inputted images, with respect to a distribution 160^{T} of the latent space encodings having common workflow data, comprises inputting the latent space encoding 150, z of the inputted images into a second neural network. The second neural network is trained to determine the one or more subsequent workflow steps to successfully treat the thrombus based on the latent space encoding 150, z of the inputted images , using training data comprising a plurality of temporal sequences of training X-ray images, and wherein each sequence represents an expansion of a stent of an intraluminal stent retriever device over a thrombus having a known value for the physical property and wherein the training data comprises workflow data for each temporal sequence of training X-ray images, the workflow data defining one or more subsequent workflow steps used to successfully treat the thrombus in the temporal sequence of training X-ray images.

With reference to the distributions 160^{T}_{0..n} illustrated in Fig. 5, by using a neural network to perform this operation, the task of determining the distance between a latent space encoding of a new temporal sequence and the centroids of the multiple distributions 160^{T}_{0..n}, is alleviated. This second neural network may be trained in a similar manner to that described above.

In some examples, the neural networks described with reference to Fig. 4 and Fig. 5 may be trained using additional data in the form of patient data. By providing this patient data as a separate input to the neural network, the neural network may learn dependencies between this data and the generated latent space encodings 150. Patient data such as age, gender, and so forth, may be inputted into the neural network during training and used to train the neural network 140. During inference, patient data may likewise be inputted into the trained neural network and used to generate the latent space encodings 150, z of the inputted images. The patient data may be extracted from the health records of patients who have undergone historic mechanical thrombectomy procedures. Similarly, volumetric images representing a vasculature surrounding the thrombus may be available for the patient, and these volumetric images may also be used during training and at inference. The volumetric images may for instance include CT images, or MRI or MRA images of the vasculature. Thus, in some examples, the neural network 140 is trained to generate the latent space encoding 150, z of the inputted images based further on patient data; and the method described with reference to Fig. 4 further includes:
receiving patient data relating to the thrombus 110;
inputting the patient data into the neural network; and
generating, using the neural network, the latent space encoding 150, z of the inputted images based further on the inputted patient data; and
wherein the patient data comprises one or more of: a volumetric image representing a vasculature surrounding the thrombus 110; electronic health record data relating to the thrombus 110.

In some examples, the neural networks described with reference to Fig. 4 and Fig. 5 may be trained using additional data in the form of extracted stent data and/or thrombus data. The stent data and/or thrombus data may be extracted from the temporal sequence of X-ray images. As with the additional patient data, by providing this data as a separate input to the neural network, the neural network may learn dependencies between this data and the generated latent space encodings 150. Thus, in some examples, the neural network 140 is trained to generate the latent space encoding 150, z of the inputted images based further on stent data and/or thrombus data; and the method described with reference to Fig. 4 further includes:
extracting the stent data and/or the thrombus data, from the temporal sequence of X-ray images;
inputting the stent data and/or the thrombus data into the neural network; and
generating, using the neural network, the latent space encoding 150, z of the inputted images based further on the inputted stent data and/or thrombus data; and
wherein the stent data comprises one or more of: a dimension, a shape, and an expansion rate of the stent over time in the temporal sequence of X-ray images; and
wherein the thrombus data comprises a dimension and/or a composition of the thrombus 110.

In these examples, the data may be extracted from the X-ray images manually, or automatically. For example, the thrombus data may be determined using image processing or computer vision algorithms to segment the clot. A neural may also be trained to extract these features from the X-ray images. The stent data may be determined using computer vision techniques or a neural network, such as for example: thresholding, region growing, template matching, level sets, active contour modelling, and neural networks such as U-Nets.

In the examples described above with reference to Fig. 4 and Fig. 5, the neural networks determine the value of a physical property of the thrombus 110 based on a position of the latent space encoding 150, z of the inputted images, with respect to a distribution 160 of latent space encodings generated from temporal sequences of training X-ray images 120^{T} representing an expansion of a stent over thrombi having a known value for the physical property.

An alternative to this technique is to predict, from the inputted images, a shape of the stent in one or more images in the temporal sequence, and to determine the value of the physical property of the thrombus 110 based on a difference DE between the predicted shape of the stent in the one or more images in the temporal sequence, and an actual shape of the stent in the one or more images in the temporal sequence.

Thus, with reference to the method illustrated in Fig. 1 in one example, the operation of determining S120 the value of the physical property of the thrombus 110, comprises:
inputting a plurality of images from the temporal sequence into a neural network 140;
predicting, from the inputted images, a shape of the stent in one or more images in the temporal sequence; and
determining the value of the physical property of the thrombus 110 based on a difference DE between the predicted shape of the stent in the one or more images in the temporal sequence, and an actual shape of the stent in the one or more images in the temporal sequence; and
wherein the neural network 140 is trained to predict the shape of the stent in the one or more images in the temporal sequence using training data comprising a plurality of temporal sequences of training X-ray images 120^{T}, and wherein each sequence represents an expansion of a stent of an intraluminal stent retriever device over a thrombus having a known value for the physical property.

This method is illustrated schematically in Fig. 6, which is a schematic diagram illustrating an example of A) the training of a neural network 140 to predict the shape of a stent from a temporal sequences of X-ray images, and B) the performing of inference with a neural network 140 that is trained to predict the shape of a stent from a temporal sequences of X-ray images, in accordance with some aspects of the present disclosure. With reference to Fig. 6B, at inference, a plurality of images from a temporal sequence 120 are inputted into the neural network. In the illustrated example, the neural network predicts from one or more images in this temporal sequence, a shape of the stent in a future image after a period Dt. The shape of the stent may alternatively be predicted in the same, inputted one or more image(s), that is, when Dt = 0. A difference, DE, is then calculated between the shape of the stent in the inputted image(s) and the predicted shape of the stent. The difference DE represents how close the value of the physical property of the thrombus 110 in the inputted image(s) is, to the value of the physical property of the thrombus 110 in the training images that were used to train the neural network.

This Fig. 6 method may be implemented by the neural networks illustrated in Fig. 4 and Fig. 5, with the difference that at inference the decoder portion of these neural networks is now required in order to calculate the difference DE. The difference DE is illustrated in the lower part of Fig. 4 and Fig. 5. This difference DE may be calculated using a loss function such as the L1 loss, L2 loss, and so forth.

This Fig. 6 method exploits the same insight as that described above for the neural networks illustrated in Fig. 4 and Fig. 5, i.e. the neural network 140 is trained using temporal sequences of training X-ray images 120^{T} that represent the expansion of a stent of an intraluminal stent retriever device over a thrombus having a known value for the physical property. Consequently, at inference, if the trained neural network is inputted with a new temporal sequence that represents an intraluminal stent retriever device over a thrombus having the same value for the physical property, the neural network will be able to accurately reconstruct the inputted image(s), and in which case there will be a small difference DE between the predicted shape of the stent in the one or more images in the temporal sequence, and the actual shape of the stent in the one or more images in the temporal sequence. By contrast, at inference, if the trained neural network is inputted with a new temporal sequence that represents an intraluminal stent retriever device over a thrombus having a different value for the physical property, the neural network will be unable to accurately reconstruct the inputted image(s), and in this case there will be a large difference DE.

The neural network illustrated in Fig. 4 and Fig. 5 may also be trained to perform this Fig. 6 method in a similar manner to that described above with reference to Fig. 4, i.e. by forcing the neural network 140 to reconstruct inputted images at inference. With reference to Fig. 6A, during training, a plurality of temporal sequences of training X-ray images 120^{T} are inputted into the neural network 140. The neural network predicts a shape of the stent in the image, or in a subsequent image. A loss, *L,* is calculated between the predicted shape of the stent, and the actual shape of the stent in the training image. The loss, *L,* is used to train the neural network. Thus, the neural network may be trained to predict the shape of the stent in one or more images in the temporal sequence, by:
receiving training data, including a plurality of temporal sequences of training X-ray images 120^{T}, and wherein each temporal sequence represents an expansion of a stent of an intraluminal stent retriever device over a thrombus having a known value for the physical property;
inputting the training data into the neural network; and
for each of a plurality of the inputted training X-ray images in a temporal sequence:
   predicting a shape of the stent in one or more images in the temporal sequence, using the neural network;
   adjusting parameters of the neural network based on a difference, *L,* between the predicted shape of the stent in the one or more images in the temporal sequence and the actual shape of the stent in the one or more images in the temporal sequence; and
   repeating the predicting and the adjusting, until a stopping criterion is met.

The training data may be provided in the same manner as described above with reference to Fig. 4 and Fig. 5. Moreover, as described in relation to the training of these neural networks, the neural network may be trained such that it places higher emphasis on data within a defined region of interest than outside the region of interest. Thus, in one example, the neural network is trained to predict the shape of the stent in the one or more images in the temporal sequence, by further:
identifying a region of interest in the temporal sequences of training X-ray images, the region of interest comprising the stent and the thrombus; and
wherein the adjusting parameters of the neural network based on a difference, *L,* between the predicted shape of the stent in the one or more images in the temporal sequence and the actual shape of the stent in the one or more images in the temporal sequence; is performed by applying a weighting to the difference, and wherein a relatively higher weighting is applied to portions of the temporal sequences of training X-ray images within the region of interest, and a relatively lower weighting is applied to portions of the temporal sequences of training X-ray images outside the region of interest.

At inference, in addition to being used to determine the value of the physical property of the thrombus 110, the difference DE may also be used to recommend one or more subsequent workflow steps for treating the thrombus 110. This may be carried out by using training data that includes workflow data defining one or more subsequent workflow steps used to successfully treat the thrombus in the temporal sequence of training X-ray images. If the difference DE is small, then the successful workflow step(s) that were used in the training data may be recommended. A neural network may also be trained to learn the association between the difference DE and the subsequent workflow step(s) that were used to successfully treat the thrombus in the temporal sequence of training X-ray images. Alternatively, the neural network illustrated in Fig. 6 may additionally also be trained to generate a distribution of latent space encodings 150, z of the inputted images, and the one or more subsequent workflow steps for treating the thrombus 110 may be recommended by determining the position of the latent space encoding of the inputted temporal sequence of X-ray images with respect to a distribution 160 of the latent space encodings having common workflow data, in the same manner as was described above with reference to Fig. 4 and Fig. 5.

The neural network may also be trained to perform this method using additional data such as patient data, stent data, and thrombus data, as was described above in relation to Fig. 4 and Fig. 5.

Thus, in one example, the neural network 140 is trained to predict the shape of the stent in the one or more images in the temporal sequence based further on patient data; and the method further comprises:
receiving patient data relating to the thrombus 110;
inputting the patient data into the neural network; and
predicting, from the inputted images, the shape of the stent in the one or more image in the temporal sequence based further on the patient data; and
wherein the patient data comprises one or more of: a volumetric image representing a vasculature surrounding the thrombus 110; electronic health record data relating to the thrombus 110.

In another example, the neural network is trained to predict the shape of the stent in the one or more images in the temporal sequence based further on stent data and/or thrombus data; and the method further comprises:
extracting the stent data and/or the thrombus data, from the temporal sequence of X-ray images;
inputting the stent data and/or the thrombus data into the neural network; and
predicting, from the inputted images, the shape of the stent in the one or more images in the temporal sequence based further on the inputted stent data and/or thrombus data; and
wherein the stent data comprises one or more of: a dimension, a shape, and an expansion rate of the stent over time in the temporal sequence of X-ray images; and
wherein the thrombus data comprises a dimension and/or a composition of the thrombus 110.

In another example, confidence values for the latent space encoding 150, z, or for the predicted shape of the stent, may be calculated. The confidence value represents a confidence of the predictions made by the neural network 140, and it permits decisions to be made based on its outputs. For example, if the confidence is low, it might be decided not to rely upon the physical properties of the thrombus that are determined. In this regard, a warning may be outputted if the confidence value is below a predetermined threshold value.

Thus, the method described with reference to Fig. 4 or Fig. 5 may additionally include the operation of:
calculating a confidence value for the latent space encoding 150, z, or for the predicted shape of the stent, respectively; and
outputting the confidence value.

By way of an example, the neural network 140 may generate the confidence value using the dropout technique. The dropout technique involves iteratively inputting the same data into a neural network and determining the neural network's output whilst randomly excluding a proportion of the neurons from the neural network in each iteration. The outputs of the neural network are then analyzed to provide mean and variance values. The mean value represents the final output, and the magnitude of the variance indicates whether the neural network is consistent in its predictions, in which case the variance is small and the confidence value is relatively higher, or whether the neural network was inconsistent in its predictions, in which case the variance is larger and the confidence value is relatively lower. The confidence may alternatively be calculated using e.g. the Kullback-Leibler "KL" divergence, between the distribution that the input image representation is sampled from and the distribution over the current trained encodings. This divergence indicates how well the input sequence is represented by the learned encodings.

In another example, a computer program product, is provided. The computer program product comprises instructions, which when executed by one or more processors, cause the one or more processors to carry out a method of determining a value of a physical property of a thrombus 110. The method comprises:
receiving S110 X-ray image data comprising a temporal sequence of X-ray images 120 representing an expansion of a stent 130 of an intraluminal stent retriever device over the thrombus 110;
determining S120 the value of the physical property of the thrombus 110 based on a rate of expansion of the stent 130 in the temporal sequence of X-ray images 120; and
outputting S130 the value of the physical property.

In another example, a system 200 for determining a value of a physical property of a thrombus 110, is provided. The system comprises one or more processors 210 configured to: receive S110 X-ray image data comprising a temporal sequence of X-ray images 120 representing an expansion of a stent 130 of an intraluminal stent retriever device over the thrombus 110;
determine S120 the value of the physical property of the thrombus 110 based on a rate of expansion of the stent 130 in the temporal sequence of X-ray images 120; and
output S130 the value of the physical property.

An example of the system 200 is illustrated in Fig. 2. In some examples, the system 200 may also include one or more of: a projection X-ray imaging system 220 for providing the temporal sequence of X-ray images 120, as illustrated in Fig. 2; an intraluminal stent retriever device 230 for performing a mechanical thrombectomy procedure on a vessel, as illustrated in Fig. 2; a display device, such as the monitor 240 illustrated in Fig. 2, for displaying the value of the physical property of the thrombus 110, the temporal sequence of X-ray images 120 representing an expansion of a stent 130 of an intraluminal stent retriever device over the thrombus 110, the one or more recommended subsequent workflow steps for treating the thrombus 110, the confidence value(s), and so forth; a patient bed 250; and a user input device for receiving user input relating to the operations performed by the system, such as a keyboard, a mouse, a touchscreen, and so forth.

The above examples are to be understood as illustrative of the present disclosure, and not restrictive. Further examples are also contemplated. For instance, the examples described in relation to computer-implemented methods, may also be provided by the computer program product, or by the computer-readable storage medium, or by the system 200, in a corresponding manner. It is to be understood that a feature described in relation to any one example may be used alone, or in combination with other described features, and may be used in combination with one or more features of another of the examples, or a combination of other examples. Furthermore, equivalents and modifications not described above may also be employed without departing from the scope of the invention, which is defined in the accompanying claims. In the claims, the word "comprising" does not exclude other elements or operations, and the indefinite article "a" or "an" does not exclude a plurality. The mere fact that certain features are recited in mutually different dependent claims does not indicate that a combination of these features cannot be used to advantage. Any reference signs in the claims should not be construed as limiting their scope.

## Claims

1. A computer-implemented method of determining a value of a physical property of a thrombus (110), the method comprising:
receiving (S110) X-ray image data comprising a temporal sequence of X-ray images (120) representing an expansion of a stent (130) of an intraluminal stent retriever device over the thrombus (110);
determining (S120) the value of the physical property of the thrombus (110) based on a rate of expansion of the stent (130) in the temporal sequence of X-ray images (120); and outputting (S130) the value of the physical property.

2. The computer-implemented method according to claim 1, wherein the determining (S120) the value of the physical property of the thrombus (110), comprises:
inputting a plurality of images from the temporal sequence into a neural network (140);
generating, using the neural network (140), a latent space encoding (150, z) of the inputted images; and
determining the value of the physical property of the thrombus (110) based on a position of the latent space encoding (150, z) of the inputted images, with respect to a distribution (160^{T}) of latent space encodings generated from temporal sequences of training X-ray images (120^{T}) representing an expansion of a stent over thrombi having a known value for the physical property; and
wherein the neural network (140) is trained to generate the latent space encoding (150, z) of the inputted images using training data comprising the temporal sequences of training X-ray images (120^{T}), and wherein each sequence represents an expansion of a stent of an intraluminal stent retriever device over a thrombus having a known value for the physical property.

3. The computer-implemented method according to claim 2, wherein the training data further comprises workflow data for each temporal sequence of training X-ray images (120^{T}), the workflow data defining one or more subsequent workflow steps used to successfully treat the thrombus in the temporal sequence of training X-ray images; and
wherein the method further comprises:
determining a position of the latent space encoding (150, z) of the inputted images, with respect to a distribution (160^{T}) of the latent space encodings having common workflow data; and
outputting the common workflow data to provide one or more recommended subsequent workflow steps for treating the thrombus (110).

4. The computer-implemented method according to claim 3, wherein the training data comprises a plurality of temporal sequences of training X-ray images having different workflow data (170^{T}_{0..n}); and
wherein the determining a position of the latent space encoding (150, z) of the inputted images, with respect to a distribution (160^{T}_{0..n}) of the latent space encodings having common workflow data, comprises determining the position of the latent space encoding (150, z) of the inputted images, with respect to a centroid of each distribution (160^{T}_{0..n}) of the latent space encodings having common workflow data; and
wherein the outputting the common workflow data to provide one or more recommended subsequent workflow steps for treating the thrombus (110), comprises outputting the common workflow data for the distribution (160^{T}_{0..n}) having the nearest centroid to the position of the latent space encoding (150, z) of the inputted images.

5. The computer-implemented method according to claim 3 or claim 4, wherein the workflow data further comprises a success metric corresponding to the one or more subsequent workflow steps; the success metric representing a probability of the one or more subsequent workflow steps resulting in a successful treatment of the thrombus (110); and
wherein the outputting the common workflow data, further comprises outputting the corresponding success metric.

6. The computer-implemented method according to claim 3, wherein the determining a position of the latent space encoding (150, z) of the inputted images, with respect to a distribution (160^{T}) of the latent space encodings having common workflow data, comprises inputting the latent space encoding (150, z) of the inputted images into a second neural network;
wherein the second neural network is trained to determine the one or more subsequent workflow steps to successfully treat the thrombus based on the latent space encoding 150, z of the inputted images, and to output the one or more subsequent workflow steps, using training data comprising a plurality of temporal sequences of training X-ray images, and wherein each sequence represents an expansion of a stent of an intraluminal stent retriever device over a thrombus having a known value for the physical property and wherein the training data comprises workflow data for each temporal sequence of training X-ray images, the workflow data defining one or more subsequent workflow steps used to successfully treat the thrombus in the temporal sequence of training X-ray images.

7. The computer-implemented method according to any one of claims 2 - 6, wherein the neural network (140) is trained to generate the latent space encoding (150, z) of the inputted images based further on patient data; and wherein the method further comprises:
receiving patient data relating to the thrombus (110);
inputting the patient data into the neural network (140); and
generating, using the neural network (140), the latent space encoding (150, z) of the inputted images based further on the inputted patient data; and
wherein the patient data comprises one or more of: a volumetric image representing a vasculature surrounding the thrombus (110); electronic health record data relating to the thrombus (110).

8. The computer-implemented method according to any one of claims 2 - 7, wherein the neural network (140) is trained to generate the latent space encoding (150, z) of the inputted images based further on stent data and/or thrombus data; and wherein the method further comprises:
extracting the stent data and/or the thrombus data, from the temporal sequence of X-ray images;
inputting the stent data and/or the thrombus data into the neural network (140); and
generating, using the neural network (140), the latent space encoding (150, z) of the inputted images based further on the inputted stent data and/or thrombus data; and
wherein the stent data comprises one or more of: a dimension, a shape, and an expansion rate of the stent over time in the temporal sequence of X-ray images; and
wherein the thrombus data comprises a dimension and/or a composition of the thrombus (110).

9. The computer-implemented method according to claim 2, wherein the neural network (140) is trained to generate the latent space encoding (150, z) of the inputted images, by:
receiving training data, including a plurality of temporal sequences of training X-ray images (120^{T}), and wherein each temporal sequence represents an expansion of a stent of an intraluminal stent retriever device over a thrombus having a known value for the physical property;
inputting the training data into the neural network (140); and
for each of a plurality of the inputted training X-ray images in a temporal sequence:
generating a latent space encoding (z) of the inputted training X-ray image, using the neural network (140);
reconstructing the inputted training X-ray image from the latent space encoding (z), using the neural network (140); and
adjusting parameters of the neural network (140) based on a difference between the inputted training X-ray image and the reconstructed inputted training X-ray image; and
repeating the generating, the reconstructing, and the adjusting, until a stopping criterion is met.

10. The computer-implemented method according to claim 9, wherein the neural network (140) is trained to generate the latent space encoding (150, z) of the inputted images, by further:
receiving input indicative of a region of interest in the temporal sequences of training X-ray images, the region of interest comprising the stent and the thrombus; and
wherein the adjusting parameters of the neural network (140) based on a difference between the inputted training X-ray image and the reconstructed inputted training X-ray image; is performed by applying a weighting to the difference, and wherein a relatively higher weighting is applied to portions of the temporal sequences of training X-ray images within the region of interest, and a relatively lower weighting is applied to portions of the temporal sequences of training X-ray images outside the region of interest.

11. The computer-implemented method according to claim 1, wherein the determining (S120) the value of the physical property of the thrombus (110), comprises:
inputting a plurality of images from the temporal sequence into a neural network (140);
predicting, from the inputted images, a shape of the stent in one or more images in the temporal sequence; and
determining the value of the physical property of the thrombus (110) based on a difference (DE) between the predicted shape of the stent in the one or more images in the temporal sequence, and an actual shape of the stent in the one or more images in the temporal sequence; and
wherein the neural network (140) is trained to predict the shape of the stent in the one or more images in the temporal sequence using training data comprising a plurality of temporal sequences of training X-ray images (120^{T}), and wherein each sequence represents an expansion of a stent of an intraluminal stent retriever device over a thrombus having a known value for the physical property.

12. The computer-implemented method according to claim 11, wherein the neural network (140) is trained to predict the shape of the stent in the one or more images in the temporal sequence based further on patient data; and wherein the method further comprises:
receiving patient data relating to the thrombus (110);
inputting the patient data into the neural network; and
predicting, from the inputted images, the shape of the stent in the one or more image in the temporal sequence based further on the patient data; and
wherein the patient data comprises one or more of: a volumetric image representing a vasculature surrounding the thrombus (110); electronic health record data relating to the thrombus (110).

13. The computer-implemented method according to any one of claims 11 - 12, wherein the neural network is trained to predict the shape of the stent in the one or more images in the temporal sequence based further on stent data and/or thrombus data; and wherein the method further comprises:
extracting the stent data and/or the thrombus data, from the temporal sequence of X-ray images;
inputting the stent data and/or the thrombus data into the neural network; and
predicting, from the inputted images, the shape of the stent in the one or more images in the temporal sequence based further on the inputted stent data and/or thrombus data; and
wherein the stent data comprises one or more of: a dimension, a shape, and an expansion rate of the stent over time in the temporal sequence of X-ray images; and
wherein the thrombus data comprises a dimension and/or a composition of the thrombus (110).

14. The computer-implemented method according to claim 11, wherein the neural network is trained to predict the shape of the stent in one or more images in the temporal sequence, by:
receiving training data, including a plurality of temporal sequences of training X-ray images (120^{T}), and wherein each temporal sequence represents an expansion of a stent of an intraluminal stent retriever device over a thrombus having a known value for the physical property;
inputting the training data into the neural network; and
for each of a plurality of the inputted training X-ray images in a temporal sequence:
predicting a shape of the stent in one or more images in the temporal sequence, using the neural network;
adjusting parameters of the neural network based on a difference (L) between the predicted shape of the stent in the one or more images in the temporal sequence and the actual shape of the stent in the one or more images in the temporal sequence; and
repeating the predicting and the adjusting, until a stopping criterion is met.

15. The computer-implemented method according to claim 2 or claim 11, further comprising:
calculating a confidence value for the latent space encoding (150, z), or for the predicted shape of the stent, respectively; and
outputting the confidence value.
